# EUROPEAN PATENT SPECIFICATION

(11) **EP 3 852 772 B1**
(45) Date of publication and mention of the grant of the patent: **23.08.2023**
(21) Application number: 19769796.4
(22) Date of filing: 17.09.2019
(51) Int. Cl.: A61K 35/28, A61P 25/16

(54) **PHARMACEUTICAL PRODUCT FOR USE IN TREATING ALZHEIMER'S DISEASE**
PHARMAZEUTISCHES PRODUKT ZUR VERWENDUNG IN DER BEHANDLUNG VON MORBUS ALZHEIMER
PRODUIT PHARMACEUTIQUE POUR L'UTILISATION DANS LE TRAITEMENT DE LA MALADIE D'ALZHEIMER

(30) Priority: 17.09.2018 ES 201830896
(43) Date of publication of application: 28.07.2021
(73) Proprietor: Fundación para la Investigación Biomédica del Hospital Universitario Puerta de Hierro Majadahonda, 28222 Majadahonda (Madrid) (ES); Fundación Mapfre, 28004 Madrid (ES); Fundación Rafael del Pino, 28010 Madrid (ES); Universidad Autónoma de Madrid, 28049 Madrid (ES)
(72) Inventor: ZURITA CASTILLO, Mercedes, 28222 Majadahonda (Madrid) (ES); VAQUERO CRESPO, Jesús, 28222 Majadahonda (Madrid) (ES)
(74) Representative: Hoffmann Eitle
(86) International application number: PCT/EP2019/074826
(87) International publication number: WO 2020/058246

(56) References cited:
- WO-A1-2007/136156
- WO-A1-2016/205371
- WO-A2-2012/156968

## Description

### Field of the invention

The present invention relates to a mesenchymal stromal cell composition for preventing or treating neurodegenerative diseases, in particular for preventing or treating Alzheimer.

### Background of the invention

Alzheimer's disease is the most common senile dementia across the world, and is characterized by progressive cognitive impairments (such as memory problems, disorientation, learning disability, and space perception impairment). In a person with Alzheimer's disease, amyloid plaques are seen in the cerebral cortex, and amyloid-β is the main component of the amyloid plaques. Various therapeutic agents for Alzheimer's disease have been proposed. Unfortunately, however, no effective therapeutic agent is currently present.

### Brief description of the invention

The present invention refers to a cell suspension comprising expanded autologous or allogeneic mesenchymal stromal cells derived from the bone marrow of a human subject, wherein said MSCs are suspended in plasma obtained from the peripheral blood from a patient or subject to be treated with the cell suspension, for use in the treatment or amelioration of a disease with cerebral impaired glucose metabolism, wherein the disease is Alzheimer's disease, wherein said cell suspension is administered to the patient or subject via intrathecal (through an intrathecal administration), in particular by lumbar puncture.

### Brief description of the figures

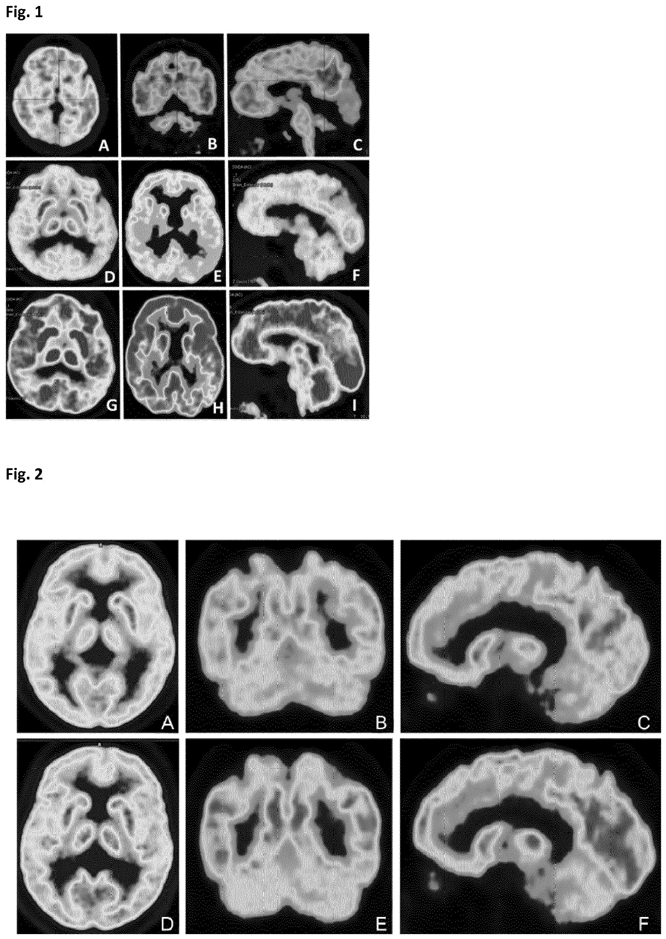

**Figure 1****.** PET studies in Case 1. A-C: Detection of beta-amyloid neuritic plaques with 18F-Flutemetamol-PET. D-E: 18F-FDG-PET previous to cell therapy. This study showed hypometabolism in left parietotemporal territory, with involvement of precuneus and posterior cingulate. A decrease in glucose captation in the mesial region and anterior poles of both temporal lobes with hippocampal territory affectation and a focal hypometabolism on the right lateral temporal territory were also observed. G-I: 18F-FDG-PET one week after the first intrathecal administration of 100 million MSCs, showing strong increase in cerebral glucose metabolism.
**Figure 2****.** Case 2. 18F-FDG-PET showing metabolic activity prior to cell therapy (AC) and at the end of treatment (DF). A clear increase in cerebral metabolism can be seen at the end of cell therapy.

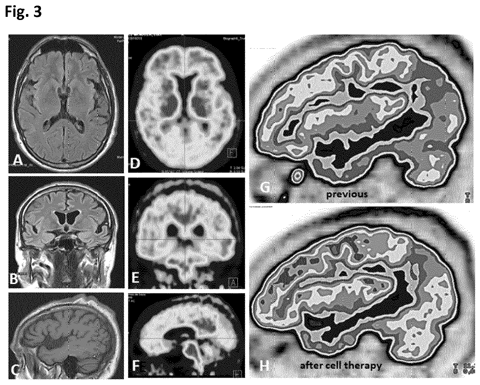

**Figure 3****.** Case 3. A-C: Magnetic Resonance (MR) showing characteristic findings of frontotemporal atrophy, predominantly on the left side. D-F: 18F-Flutemetamol-PET for detection of beta-amyloid neuritic plaques. G: (18F-FDG-PET) performed previous to cell therapy. H: (18F-FDG-PET) at the end of cell therapy. At this time, strong increase of cerebral glucose metabolism can be seen.

### Description of the invention

### DEFINITIONS

As used herein, the following terms and phrases shall have the meanings set forth below. Unless defined otherwise, all technical and scientific terms used herein have the same meaning as commonly understood to one of ordinary skill in the art to which this invention belongs.

The articles "a" and "an" refer to one or to more than one (i.e., to at least one) of the grammatical object of the article. By way of example, "an element" means one element or more than one element.

As used herein "autologous" is understood as referring to a cell preparation where the donor and the recipient are the same individual.

As used herein "allogeneic" is understood as referring to a cell preparation where the donor and the recipient are not the same individual.

As used herein "adult bone marrow derived cells" is understood as a preparation comprising cells, which are not embryonic and are derived from bone marrow obtained from a human donor.

As used herein "cell suspension" is understood as a preparation of cells suspended in a liquid medium.

"Mesenchymal stromal cells (also referred to herein as "MSCs") are multipotent stromal cells, i.e. they are cells which are capable of giving rise to multiple different types of cells.

The term "expanded" as used herein when referring to cells shall be taken to have its usual meaning in the art, namely cells that have been proliferated in vitro. A MSC can be expanded to provide a population of cells that retain at least one biological function of the MSC, typically the ability to adhere to a plastic surface, under standard culture conditions. The expanded population of cells may retain the ability to differentiate into one or more cell types.

As used herein "cell suspension of adult MSCs bone marrow derived cells" is understood as a preparation of expanded mesenchymal stromal cells obtained from bone marrow and suspended in, preferably autologous, plasma, which are not embryonic and are derived from bone marrow obtained from a human donor, subject or patient. As indicated, such cell suspension of adult bone marrow derived cells consists of mesenchymal stromal cells and plasma as its excipient, an illustrative and non-limiting manner of preparing said cell suspension is fully described in example 1.

As used herein, "intrathecal" refers to the intrathecal space of a vertebrate spine, typically a mammalian spine, and often a human spine. The intrathecal space is defined as the space within the sheath of dura matter surrounding the spinal cord. The intrathecal space (subarachnoid space) is occupied by cerebrospinal fluid.

"Support" as used herein refers to any device or material that may serve as a foundation or matrix for the growth of bone marrow tissue-derived stromal stem cells.

As used herein, "treatment" should be understood to include any indicia of success in the treatment, alleviation or amelioration of an injury, pathology or condition. This may include parameters such as abatement, remission, diminishing of symptoms; slowing in the rate of degeneration or decline; making the final point of degeneration less debilitating; improving a patient's physical or mental well-being; or, in some situations, reducing the likelihood of onset of disease.

As used herein, the terms "patient" and "subject" are used interchangeably and refer to human or other mammalian patients and subjects and includes any individual it is desired to examine or treat using the methods of the invention. However, it will be understood that "patient" does not imply that symptoms are present. Suitable mammals that fall within the scope of the invention include, but are not restricted to, primates, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes).

As used herein, the term "neurodegenerative disease" refers to a disease characterized by a progressive decline in the structure, activity, and/or function of neural tissue, including brain tissue. Neurodegenerative diseases include, but are not limited to, Alzheimer's disease, Parkinson's disease, amyotrophic lateral sclerosis (ALS), Huntington's disease, Friedreich's ataxia, frontotemporal lobar degeneration, and dementia with Lewy bodies.

As used herein, the term "progression of a neurodegenerative disease" refers to the gradual worsening of the disease over time, whereby symptoms and neurochemical deficits become increasingly more debilitating and/or intense. Neurodegenerative disease progression often correlates to a decline in the structure, activity, and/or function of brain tissue.

The term "about" in reference to a numeric value means +/- 20% of that numeric value. The term "about" in reference to a numeric value also includes +/- 10% of that numeric value. The term "about" in reference to a numeric value also includes +/- 5% of that numeric value. The term "about" in reference to a numeric value also includes +/-1% of that numeric value.

The terms "comprise" and "comprising" are used in the inclusive, open sense, meaning that additional elements may be included. The term "comprises" also encompasses and may be used interchangeably with the terms "consists of" and "consists essentially of".

As used herein the term "adult" it is meant that the stem cells are not embryonic. In one embodiment, "adult" means post-embryonic or "post-natal". With respect to the stem cells of the present invention, the term "adult stem cell" means that the stem cell is isolated from a tissue or organ of an animal at a stage of growth later than the embryonic stage. Adult stem cells are unlike embryonic stem cells, which are defined by their origin, the inner cell mass of the blastocyst. Adult stem cells according to the invention may be isolated from any non-embryonic tissue, and will include neonates, juveniles, adolescents and adult subjects. Generally, the stem cell of the present invention will be isolated from a non-neonate mammal, and for example from a non-neonate human. Preferably, the stem cells of the present invention are isolated from a human.

The term "isolated" indicates that the cell or cell population to which it refers is not within its natural environment. The cell or cell population has been substantially separated from surrounding tissue.

### DETAILED DESCRIPTION

The present invention is defined by the claims.

In the examples of the present specification we show three clinical cases as examples of patients in early stages of Alzheimer's disease that experienced the arrest of clinical deterioration, or even clear improvement of their symptoms after being treated by intrathecal injections of autologous MSCs.

None of them received any medication for its disease at the time of receiving cell therapy. In the three cases, improvements were associated with neuropsychological improvements and with an increase in cerebral glucose metabolism, studied by 18F-FDG-PET. Although in the literature cell therapy has been considered a possible strategy in AD through neuroprotection and paracrine actions, until the present invention there was no evidence of its possible clinical usefulness. In this sense, as shown in the examples, the relationship between an increase of cerebral glucose metabolism and intrathecal administrations of autologous MSCs supported in autologous plasma offers a new line of treatment in the field of cell therapy applied to AD, mainly taking into account that the impaired glucose metabolism in the brain of subjects with AD is a commonly recognized early feature of this disease.

Our findings although preliminary and although they should be confirmed in a larger number of patients, made our findings plausible. Moreover, as described in example 2, the effects of our proposed treatment with the intrathecal administration of MCs seem to have a medium term effect. At any rate, the intrathecal administration of autologous MSCs is safe.

### THE CELL SUSPENSION PRODUCT

The cell suspension according to the present invention is a product suitable for the treatment or amelioration of a disease with cerebral impaired glucose metabolism, wherein the disease is Alzheimer's disease.

In this sense, it has been surprisingly found that the cell suspension according to the present invention when used in the treatment or amelioration of a disease with cerebral impaired glucose metabolism, namely AD, via intrathecal (or through an intrathecal administration), in particular by lumbar puncture, leads to many clinically relevant improvements such as favorable changes in the Neuropsychological studies included Mini Mental State Examination (MMSE) scale, Rey Complex Figure Test (CRFT), Stroop test, Spanish adaptation to California Verbal Learning Test (CVLT), and the Wechler Adult Intelligence Scale (WAIS-III).. All the above advantages can be obtained even with a single administration of the cell suspension and are clearly illustrated in example 2.

Generally, the cell suspension of the invention refers to a cell suspension of autologous or allogeneic, preferably autologous, adult bone marrow derived cells, preferably derived from the crest of the ilium (or iliac crest), which comprises expanding said mesenchymal stromal cells obtained from bone marrow and suspending the same in, preferably autologous, plasma. As indicated, such cell suspension of adult bone marrow derived cells consists of mesenchymal stromal cells and plasma as its excipient, an illustrative and non-limiting manner of preparing said cell suspension is fully described in example 1.

In a preferred embodiment, the cell suspension of the invention, in the final formulation, as defined in the precedent paragraph, comprises from about 30 × 10⁵ to about 30 × 10⁷, more preferably from about 1 × 10⁶ to about 100 × 10⁶, still more preferably about 30 × 10⁶ expanded MSCs obtained from bone marrow and supported in, preferably autologous, plasma, and all dosage values between each of the above listed values.

It is noted that such cell suspension can comprise, apart from plasma, further pharmaceutically acceptable carriers and/or excipients. Pharmaceutically acceptable carriers and diluents include saline, aqueous buffer solutions, and/or dispersion media. The use of such carriers and diluents is well known in the art. The solution is sterile and of sufficiently low viscosity to allow use of a syringe for administration.

Examples of materials and solutions which can serve as pharmaceutically-acceptable carriers are also well known in the art.

It is noted that in a preferred embodiment the final pharmaceutical formulation solution of the cell suspension product as illustrated in the examples of the present specification can be packaged in sterile syringes or microsyringes, such as endotoxins-free 1-mL Hamilton microsyringes with a 20-guage needle. Subsequently the needle can be removed and a sterile luer plug nut can be placed on the end of each preloaded syringe. Microsyringes with the medicament were placed inside a sterile metal box, which was also double bagged before being transported to the operating room for cell transplantation.

In another preferred embodiment, the cell suspension of the invention or the pharmaceutical composition of the invention may be frozen in freezing medium. Any medium that preserves the viability of the cells at temperatures between -135°C and -190°C is suitable as freezing medium. For example, the freezing medium may comprise 2.5% to 10% DMSO. More specifically, the freezing medium may comprise 5-10% DMSO.

After thawing, cells of invention can be washed to remove the DMSO or other freezing medium components before administration or re-suspension in plasma.

The pharmaceutical compositions of the invention may also be used in any of the methods of treatment or therapeutic uses described herein.

It is noted that the present invention further encompasses 1-3 (pre-filled) syringes comprising the cell suspension of the invention.

In addition, if desired, the cells of the cell suspension of the invention of the invention can be modified genetically by any conventional method including, by way of illustration but non-limiting, processes of transgenesis, deletions or insertions in their genome, etc.

### - MANUFACTURING PROCESS OF THE CELL SUSPENSION PRODUCT

The cell suspension product of the invention can be manufactured in a number of ways; however, they are preferably manufactured as detailed in example 1.

The manufacturing process as described in example 1 is briefly summarized as follows:
For obtaining the plasma, preferably as a first step in the preparation of the cell suspension, peripheral blood from each patient or subject must be obtained. Such blood is then centrifuged, preferably at 900g for 8 min to obtain the plasma fraction, which is then stored in cold conditions, preferably by using tubes and stored at -80°C, until the medicament formulation. Preferably then, bone marrow is aspirated under aseptic conditions, preferably from the iliac bones of each patient or subject, then anticoagulated and culture and expanded. For this purpose, mononuclear cells (MNC) are separated, preferably by density gradient using an automated cell-processing system. Then, they are plated at a density of 16 × 10⁴ to 20 × 10⁴ cells/cm². The cultures are then maintained, after which nonadherent cells are removed by replacing the medium. When the cultures approached confluence, adherent cells are detached. Then, cells are cultured to obtain the required number according to the plan previously made for each patient. Cells are then re-plated at a density of 3000-5000 cells/cm², and the culture is maintained renewing the medium every 3-4 days until a confluence of 90-100%. Once the culture reached confluency, it is prepared to obtain the bulk of mesenchymal stromal cells (MSCs). At this time, MSCs are detached. After that, MSCs are re-suspended with the previously obtained autologous plasma to remove traces of the washing medium.

After formulation, the cell therapy medicament can be packaged in sterile syringes.

Based on the above, a further aspect of the invention refers to a manufacturing process of the cell suspension of the invention, which comprises:
1. Bone marrow (BM) collection is performed by repeated aspirations, preferably from the subjects' posterior iliac crest under local or general anesthesia.
2. From such bone marrow collection, MSCs are obtained and expanded to obtain the required number according to the plan previously made for each patient thus providing a bulk of mesenchymal stromal cells (MSCs).
3. Before, simultaneously or after the bone marrow collection, peripheral blood from each patient or subject must be obtained. Such blood is then centrifuged, preferably at 900g for 8 min to obtain the plasma fraction, which is then stored in cold conditions, preferably by using tubes and stored at -80°C, until the medicament formulation.
4. Finally, MSCs are re-suspended with the obtained plasma to remove traces of the washing medium, in order to provide the cell suspension product. Such product can be later on packaged in sterile syringes

### - APPLICABILITY OF THE CELL SUSPENSION PRODUCT

Results clearly illustrated in example 2 showed that within the first 12 months following the administration of cell suspension of the invention, subjects achieved a clinically relevant response as demonstrated by the favorable changes in the Neuropsychological studies included Mini Mental State Examination (MMSE) scale, Rey Complex Figure Test (CRFT), Stroop test, Spanish adaptation to California Verbal Learning Test (CVLT), and the Wechler Adult Intelligence Scale (WAIS-III). All the above advantages can be obtained even with a single administration of the cell suspension and are clearly illustrated in example 2. In addition, example 3 shows, following the administration of cell suspension of the invention, a clinically relevant response in the neurological sequelae after a hemorrhagic stroke.

No subject experienced a Suspected Unexpected Serious Adverse Reaction (SUSAR) during the treatment of during follow-up. No subject discontinued the trial prematurely as a result of an AE and no subjects experienced an AE related to the cell suspension of the invention. Therefore, it is thus clear that the cell suspension of the invention is especially advantageous in the treatment of a disease with cerebral impaired glucose metabolism, namely AD, via intrathecal (or through an intrathecal administration), in particular by lumbar puncture.

Hence, a further aspect of the invention refers to the cell suspension, a pre-filled syringe comprising said cell suspension or a pharmaceutical composition comprising said cell suspension for use in the treatment or amelioration of a disease with cerebral impaired glucose wherein the disease is Alzheimer's disease.

Importantly, the cell suspension, the pre-filled syringe or the pharmaceutical composition, must be used for the treatment of the above mentioned disease, via intrathecal (through an intrathecal administration in the cerebrospinal fluid of the subarachnoid space), in particular by lumbar puncture.

The following examples serve to illustrate the present invention but do not limit the same.

### Examples

### Example 1. Preparation of cell suspension of adult MSCs bone marrow derived cells

For obtaining the plasma, as a first step in the preparation of the cell suspension, peripheral blood from each patient or subject must be obtained. Such blood is then centrifuged at 900g for 8 min to obtain the plasma fraction, which is aliquoted in 15 mL tubes and stored at -80°C until the medicament formulation. Then, approximately 50 mL of bone marrow is aspirated under aseptic conditions from the iliac bones of each patient or subject, immediately anticoagulated by a 5 mL solution composed of 100 IU/mL sodium heparin Chiesi (Chiesi España) and 104 IU/104 µg penicillin-streptomycin (BioWhittaker-Lonza) and sent to a cleanroom for culture and expansion under good manufacturing practice (GMP). Mononuclear cells (MNC) are separated by density gradient using an automated cell-processing system (SEPAX, BioSafe). Then, they are plated at a density of 16 × 10⁴ to 20 × 10⁴ cells/cm², in 175 cm² flasks on Alpha-Minimum Essential Medium (MEM) with Earle's Balanced Salt Solution (BSS), and supplemented with 20% fetal bovine serum (FBS), 200 mmol/L L-glutamine (BioWhittaker-Lonza) and 104 IU/104 µg penicillin-streptomycin (BioWhittaker-Lonza). The cultures are maintained at 37°C in a humidified 5% CO₂ atmosphere for 3 days, after which nonadherent cells are removed by replacing the medium. When the cultures approached confluence (90-100%), adherent cells are detached by treatment with trypsin/ethylenediamine tetraacetic acid (EDTA) solution (BioWhittaker-Lonza). Neutralization of trypsin and subsequent washing is performed with Alpha-MEM medium supplemented with 10% FBS and 2 mmol/L L-glutamine, centrifuging at 1250 rpm for 10 min. After study of viability, cells are cultured to obtain the required number according to the plan previously made for each patient. Cells are re-plated at a density of 3000-5000 cells/cm2 in factory farming of 4 floors with free-antibiotic Alpha-MEM medium supplemented with 10% FBS and 2 mmol/L L-glutamine, and the culture is maintained renewing the medium every 3-4 days until a confluence of 90-100%. Once the culture reached confluency, it is prepared to obtain the bulk of mesenchymal stromal cells (MSCs). At this time, MSCs are detached with trypsin/EDTA and washed with Hank's BSS medium (BioWhittaker-Lonza) supplemented with 5% albumin (20% albumin, Grifols). After that, MSCs are re-suspended with the previously obtained autologous plasma to remove traces of the washing medium. After cell counting, MSCs for a second dose can be separated and then cryopreserved, in a FBS solution, in dimethylsulphoxide (DMSO; Miltenyi Biotec). For this, we used a liquid nitrogen-free controlled rate freezer (EF 600, Grant-Asymptote). Finally, the MSCs for surgical administration are formulated, according to the number scheduled for each patient, after a new centrifugation at 1250 rpm for 10 min. After formulation, the cell therapy medicament can be packaged in sterile and endotoxins-free 1-mL Hamilton microsyringes, with a 20-guage needle. Subsequently the needle can be removed and a sterile luer plug nut was placed on the end of each preloaded syringe. Microsyringes with the medicament were placed inside a sterile metal box, which was also double bagged before being transported to the operating room for cell transplantation.

### Example 2. Intrathecal administration of autologous bone marrow mesenchymal stromal cells as an approach to the treatment of Alzheimer's disease

### Methods. Cell therapy medicament and Study design

The present study was conducted in accordance with the principles of the Declaration of Helsinki and good clinical practice guidelines. In three patients, informed consent was obtained.

NC1 is the cell suspension of adult MSCs bone marrow derived cells of example 1. NC1 is a cell therapy medicament developed in the cleanroom of the neurosurgical service of the Puerta de Hierro-Majadahonda Hospital, and currently approved as a medicament under clinical investigation (PEI No. 12-141). It consists of expanded autologous MSCs obtained from bone marrow, and autologous plasma as its excipient. Previous to NC1 preparation, a sample of peripheral blood was retrieved from each patient for genomic studies in order to rule out chromosomal abnormalities that could discourage cell expansion, and to obtain a genetic fingerprint (KaryoNIM Stem Cells and KaryoNIM STR test, respectively, NIMGenetics, Madrid, Spain). Techniques for culture and expansion of MSCs, phenotypic characterization, formulation and packaging have been previously described [Vaquero J, Zurita M, Rico MA, Bonilla C, Aguayo C, Fernández C, et al. Repeated subarachnoid administrations of autologous mesenchymal stromal cells supported in autologous plasma improve quality of life in patients suffering incomplete spinal cord injury. Cytotherapy 2017;19:349-59. And Vaquero J, Zurita M, Rico M A, Bonilla C, Aguayo C, Montilla J, et al. An approach to personalized cell therapy in chronic complete paraplegia: The Puerta de Hierro phase I/II clinical trial. Cytotherapy 2016;18:1024-35.].

### Clinical assessment

Clinical and neuroimage studies were performed in patients. Neuropsychological studies included Mini Mental State Examination (MMSE) scale, Rey Complex Figure Test (CRFT), Stroop test, Spanish adaptation to California Verbal Learning Test (CVLT), and the Wechler Adult Intelligence Scale (WAIS-III).

### Report of Cases

Case 1. A 65 year-old male, with more than 20 years of formal education and family history of AD (Alzheimer's disease), was diagnosed with AD 2 years before coming to our hospital to evaluate cell therapy treatment. At this moment, he described a slow but progressive neurological deterioration. In July 2017, he stopped taking his medication (donepezil, 5 mg / day). Cell therapy was administered between November 2017 and May 2018 (100 million MSCs every 3 months). At the end of treatment, the patient and his family considered that he was stable, with no progression. A neuropsychological assessment was performed before the treatment (pre) and 2 weeks after each MSC dose (post 1, post 2 and post 3). The results showed a slight improvement in MMSE: pre, 27 points; post 1, 25; post 2, 28; and post 3, 27; NAP = small effect. Short-term visual memory reached 12 points at the end of cell therapy (post 3), being 9.5 points previous, 8 points at post 1 and 8.5 points at post 2. In addition, there were statistically significant improvements on long term visual memory: pre, 6.5 points; post 1, 6 points; post 2, 8.5 points; and post 3, 8.5 points; NAP = medium effect. Mental processing speed showed the following: pre, 33 points; post 1, 38 points; post 2, 39 points; and post 3, 37 points; NAP = strong effect. Inhibition of automatic response showed the following: pre, 49 points; post 1, 48 points; post 2, 57 points; and post 3, 54 points; NAP = medium effect.. Inhibition of automatic response showed 49 points previous to cell therapy and 57 points at the end of the treatment.

Studies with 18F-FDG-PET showed progressive increases in global brain glucose metabolism during the cell therapy (Figure 1).

Case 2. A 56-year old male, with 20 years of formal education, and no family history of dementia, was diagnosed with AD 2 years before coming to our hospital to evaluate cell therapy treatment. The patient had not accepted medication for his cognitive problems, except vitamin supplements. He related progressive difficulties in his work, showing problems to remember how to organize simple activities or manage easy tasks in his computer. He also had exhibited several spatial disorientation episodes.

He received intrathecal cell therapy between November 2017 and June 2018 (100 million MSCs every 3 months). After finishing cell therapy, the patient mentioned an appreciable improvement in his daily activities, specially using his computer and with no other disorientation episodes. A neuropsychological assessment was performed before the treatment (pre) and 2 weeks after every MSC dose (post 1, post 2 and post 3). Neuropsychological examination showed improvement in MMSE (28 points, 27 points, 27 points and 30 points at prior, post 1, post 2 and after cell therapy, respectively; NAP = small effect). Verbal learning showed the following: pre, 38 points; post 1, 59 points; post 2, 54 points; and post 3, 59 points; NAP = strong effect. Verbal short-term memory test showed the following: pre, 12 points; post 1, 14 points; post 2, 14 points; and post 3, 14 points; NAP = strong effect. Verbal long-term memory test [15] showed the following: pre, 10 points; post 1, 13 points; post 2, 13 points; and post 3, 14 points; NAP = strong effect. Attention showed the following: pre, 12 points; post 1, 13 points; post 2, 16 points; and post 3, 15 points; NAP = strong effect. Mental processing speed showed the following: pre, 56 points; post 1, 59 points; post 2, 73 points; and post 3, 72 points; NAP = strong effect. Study of the inhibition of automatic response showed the following: pre, 50 points; post 1, 46 points; post 2, 49 points; and post 3, 57 points; NAP = small effect.

18F-FDG-PET showed progressive increases in global brain glucose metabolism during cell therapy (Figure 2).

Case 3. A 63-year-old male, with more than 20 years of formal education, and maternal familiy history of AD, was diagnosed at the age of 61 years with dementia due to basal frontotemporal atrophy. He presented important memory problems and disorientation episodes that had caused him to stop working. He had rejected donopezil.

Cell therapy was administered between November 2016 and May 2017 (100 million MSCs every 3 months). After first administration, the patient experienced an amelioration, especially in orientation, corroborated by his family. The benefit was progressive, and at the end of the treatment, the patient could manage his fiscal obligations and going out without any disorientation problems. The family also reported better communication skills and evident greater participation in interactions with family or friends. The patient showed a score of 30 on MMSE prior to the treatment and maintained it at the end of treatment. Neuropsychological assessment was performed before the treatment (pre) and 2 weeks after each MSCs administration (post 1, post 2 and post 3). The data showed a statistically significant improvement in verbal learning: pre, 28 points; post 1, 32 points; post 2, 36 points; and post 3, 40 points; NAP = strong effect. Verbal short-term memory showed the following: pre, 5 points; post 1, 6 points; post 2, 5 points; and post 3, 7 points; NAP =medium effect. Verbal long-term memory showed the following: pre, 4 points; post 1, 8 points; post 2, 5 points; and post 3, 8 points; NAP = strong effect. The study of semantic memory showed the following: pre, 25 points; post 1, 27 points; post 2, 26 points; and post 3, 29 points; NAP = strong effect. Working memory showed the following: pre, 5 points; post 1, 10 points; post 2, 11 points; and post 3, 12 points; NAP = strong effect. Study of inhibition showed the following: pre, 47 points; post 1, 54 points; post 2, 49 points; and post 3, 58 points; NAP = strong effect.

In the course of cell therapy, injections of MSCs induced a progressive increase in brain glucose metabolism (Figure 3).

In May 2018, one year after the end of the cell therapy, a new assessment was made. At this time, MMSE score was 26, and the patient showed worse scores on verbal short-term memory that decreases to 2 points, but he got the same scores on verbal learning, and long-term memory. Furthermore, he presented a better performance on semantic memory, working memory, and inhibition. On the other hand, at this time, brain glucose metabolism decreased when compared with the 18F-FDG-PET scan obtained at the end of treatment, but the patient maintained the clinical improvement that manifested in May 2017.

### Example 3. Late Cell Therapy with Intrathecal Autologous Mesenchymal Stromal Cells Increases Brain Glucose Metabolism and Improves the Sequelae of Cerebral Hemorrhage

### Case Report

A 49 year-old female, right-handed, and computer engineer, with more than 20 years of formal education, suffered in 2009 from spontaneous intracerebral hemorrhage in the left frontoparietal area, open to ventricule. Angiographic study did not show presence of aneurysms or vascular malformations. After spontaneous resolution of the hemorrhage, the patient has been receiving a comprehensive neurorehabilitation program of physiotherapy, occupational therapy, speech therapy and neuropsychological intervention. Currently, she continues her physiotherapy and neuropsychological treatment.

In 2017, 8 years after brain hemorrhage, the patient comes to us requesting a possible treatment of cell therapy. At this time a study of Magnetic Resonance (MR) showed a large intracerebral cavity in the area where the hemorrhage took place, communicated with the ventricular system, and 18F-FDG-PET showed hypometabolism in the areas adjacent to the lesion, and in posterior region of the left basal ganglia.

Clinical examination showed residual right hemiparesis (great difficulty in gait, loss of balance and instability requiring the use of external supports), and severe spasticity on right upper extremity, especially on hand, with no functionality. She complained of communication problems and cognitive deficits such as memory problems, attention difficulties (mental fatigue after few minutes using the computer, difficulties to follow conversations, to attend several stimuli at the same time), anomia, reading problems (she hardly could read half page without forgetting it or be feeling very tired). Neuropsychological assessment revealed a poor performance, mainly in attention, learning, and memory, planning, naming, verbal fluency, speech and gesture apraxia tasks.

Spanish Agency for Medications and Health Products (AEMPS), and Medical Management of Puerta de Hierro-Majadahonda Hospital authorized us the treatment with our NC1 medicament. As already indicated, this medicament consists of expanded autologous MSCs obtained from bone marrow, and autologous plasma as its excipient.

Between March 2017 and September 2017, the patient received 3 intrathecal administrations of 100 million de autologous mesenchymal stromal cells each three months, until a total dose of 300 million of MSCs. This treatment study conducted in accordance with the principles of the Declaration of Helsinki 10 and good clinical practice guidelines. Informed consent was obtained, after explaining the experimental nature of the treatment and our previous experience obtained with intrathecal administration of this type of cell therapy in patients with neurological sequelae due to spinal cord injury (SCI) and traumatic brain injury (TBI).

### RESULTS

From the first administration of cell therapy, the patient reported progressively a generalized and appreciable improvement in her mobility and cognitive deficits. At the end of the treatment, a clear reduction in spasticity and improvement in her stability and gait was observed. She can walk for more than one hour without external supports or supervision of her movements. Attention and planning abilities got better. She can work on her computer for several hours without feeling tired, even when she is doing complex tasks. She also enjoys reading for at least one hour, remembering most of it. Articulation of language and oral expression have also improved, and autonomy for daily activities is also increased. Furthermore, prior to cells transplantation, the patient described taking laxatives and a high-fiber diet for constipation. Just after the first cell doses, she experienced no need of them. Her family, friends, physiotherapist, and neuropsychologist corroborated all these advances.

Neuropsychological assessment confirmed cognitive improvement after cell therapy. She showed a better performance on Addenbrooke Cognitive Examination-Revised (ACE-R) test, with 94 points (79 points previous). Visual and verbal memory, measured by Rey Complex Figure Test (RCFT), and the Spanish adaptation (TAVEC) of California Verbal Learning Test (CVLT) respectively, also increased notably.

Furthermore, the patient had a clear enhancement in planning (Zoo test profile score from -1 to 3), working memory (10 to 12) measured by Letters and Numbers test, naming 11 to 12 correct spontaneous words, 4 to 3 phonetic cues on Short-Form Boston Naming Test (BNT), and semantic fluency (animals, vegetables, kitchen utensils and clothes), with mean score from 12.25 to 15. These clinical improvements are maintained one year after finishing MSCs administration.

Studies with 18F-FDG-PET performed approximately two weeks after each administration of MSCs showed progressive increases in global brain glucose metabolism during the cell therapy .

### CONCLUSIONS

Our present case shows the possibility of using intrathecal cell therapy with autologous MSCs to alleviate chronically established neurological sequelae after a hemorrhagic stroke. The finding of a progressive increase in brain glucose uptake in the course of cell therapy, suggests that intrathecal MSCs can reactivate brain metabolism, contributing to permanent clinical improvement.

## Claims

1. A cell suspension comprising expanded autologous or allogeneic mesenchymal stromal cells (MSCs) derived from the bone marrow of a human subject, wherein said MSCs are suspended in plasma obtained from the peripheral blood from a patient or subject to be treated with the cell suspension, for use in the treatment or amelioration of a disease with cerebral impaired glucose metabolism, wherein said cell suspension is administered to the patient via intrathecal (through an intrathecal administration in the cerebrospinal fluid of the subarachnoid space), in particular by lumbar puncture, and wherein the disease with cerebral impaired glucose metabolism is Alzheimer's disease.

2. The cell suspension for use according to claim 1, wherein the MSCs are autologous.

3. The cell suspension for use according to any of the precedent claims, wherein the cell suspension comprises from about 30 × 10⁵ to about 30 × 10⁷, more preferably from about 1 × 10⁶ to about 100 × 10⁶, still more preferably about 30 × 10⁶ expanded MSCs obtained from bone marrow.

4. A syringe or a plurality of syringes comprising a cell suspension as defined in any of claims 1 to 3, for use in the treatment or amelioration of a disease with cerebral impaired glucose metabolism, wherein said cell suspension is administered to the patient via intrathecal (through an intrathecal administration), in particular by lumbar puncture, and wherein the disease with cerebral impaired glucose metabolism is Alzheimer's disease.

5. The cell suspension for use according to any one of claims 1-3 or the syringe(s) for use according to claim 4, wherein said suspension is provided as a single dose or as multiples dosages.

## Patentansprüche

1. Zellsuspension umfassend expandierte autologe oder allogene mesenchymale Stromazellen (MSC) abgeleitet vom Knochenmark eines menschlichen Individuums, wobei die genannten MSC in Plasma suspendiert werden, erhalten aus dem peripheren Blut eines Patienten oder Individuums, welcher/welches mit der Zellsuspension behandelt werden soll, für deren Verwendung in der Behandlung oder Linderung einer Krankheit mit beeinträchtigtem Hirnglukosestoffwechsel, wobei die genannte Zellsuspension dem Patienten intrathekal (über eine intrathekale Verabreichung in der Gehirn-Rückenmark-Flüssigkeit des Subarachnoidalraums), insbesondere mittels Lumbalpunktion verabreicht wird, und wobei die Krankheit mit beeinträchtigtem Hirnglukosestoffwechsel Morbus Alzheimer ist.

2. Zellsuspension für deren Verwendung nach Anspruch 1, wobei die MSC autolog sind.

3. Zellsuspension für deren Verwendung nach einem der vorhergehenden Ansprüche, wobei die Zellsuspension von etwa 30 × 10⁵ bis etwa 30 × 10⁷, weiter vorzugsweise von etwa 1 × 10⁶ bis etwa 100 × 10⁶, noch weiter vorzugsweise etwa 30 × 10⁶ expandierte MSC erhalten aus Knochenmark umfasst.

4. Spritze oder Vielzahl von Spritzen umfassend eine Zellsuspension nach einem der Ansprüche 1 bis 3, für deren Verwendung in der Behandlung oder Linderung einer Krankheit mit beeinträchtigtem Hirnglukosestoffwechsel, wobei die genannte Zellsuspension dem Patienten intrathekal (über eine intrathekale Verabreichung), insbesondere mittels Lumbalpunktion verabreicht wird, und wobei die Krankheit mit beeinträchtigtem Hirnglukosestoffwechsel Morbus Alzheimer ist.

5. Zellsuspension für deren Verwendung nach einem der Ansprüche 1-3 oder Spritze(n) für deren Verwendung nach Anspruch 4, wobei die genannte Suspension als Einzeldosis oder als mehrfache Dosierungen bereitgestellt wird.

## Revendications

1. Suspension cellulaire comprenant des cellules stromales mésenchymateuses (CSM) expansées, autologues ou allogènes, provenant de la moelle osseuse d'un sujet humain, dans laquelle lesdites CSM sont suspendues dans du plasma obtenu depuis le sang périphérique d'un patient ou sujet à traiter avec la suspension cellulaire, pour l'utilisation dans le traitement ou l'amélioration d'une maladie avec déficience du métabolisme cérébral du glucose, dans laquelle ladite suspension cellulaire est administrée au patient par voie intrathécale (par une administration intrathécale dans le liquide cérébro-spinal de l'espace sous-arachnoïdien), en particulier par ponction lombaire, et dans laquelle la maladie avec déficience du métabolisme cérébral du glucose est maladie d'Alzheimer.

2. Suspension cellulaire pour l'utilisation selon la revendication 1, dans laquelle les CSM sont autologues.

3. Suspension cellulaire pour l'utilisation selon l'une des revendications précédentes, dans laquelle la suspension cellulaire comprend d'environ 30 × 10⁵ jusqu'à environ 30 × 10⁷, plus préférablement d'environ 1 × 10⁶ jusqu'à environ 100 × 10⁶, encore plus préférablement environ 30 × 10⁶ CSM expansées obtenues de la moelle osseuse.

4. Seringue ou pluralité de seringues comprenant une suspension cellulaire selon l'une quelconque des revendications 1 à 3, pour l'utilisation dans le traitement ou l'amélioration d'une maladie avec déficience du métabolisme cérébral du glucose, dans laquelle ladite suspension cellulaire est administrée au patient par voie intrathécale (par une administration intrathécale), en particulier par ponction lombaire, et dans laquelle la maladie avec déficience du métabolisme cérébral du glucose est maladie d'Alzheimer.

5. Suspension cellulaire pour l'utilisation selon l'une quelconque des revendications 1 à 3, ou seringue(s) pour l'utilisation selon la revendication 4, dans lesquelles ladite suspension est fournie comme une dose unique ou comme multiples dosages.
